Europäisches Patentamt

European Patent Office

Office européen des brevets

(11) Veröffentlichungsnummer: **0 353 185**
**A1**

## EUROPÄISCHE PATENTANMELDUNG

(21) Anmeldenummer: **89810528.3**

(22) Anmeldetag: **12.07.89**

(51) Int. Cl.5: **A 61 F 13/32**

(30) Priorität: **20.07.88 CH 2784/88**

(43) Veröffentlichungstag der Anmeldung:
**31.01.90 Patentblatt 90/05**

(84) Benannte Vertragsstaaten:
**AT BE CH DE ES FR GR IT LI LU NL SE**

(71) Anmelder: **Fassbind & Fassbind**
**Appisbergstrasse 20**
**CH-8708 Männedorf (CH)**

(72) Erfinder: **Fassbind, Karl**
**Appisbergstrasse 20**
**CH-8708 Männedorf (CH)**

(74) Vertreter: **Justitz-Wormser, Daisy P., Dipl.-Chem. et al**
**c/o Patentanwalts-Buraeu ISLER AG Walchestrasse 23**
**CH-8006 Zürich (CH)**

(54) Vorrichtung zur Einführung fester, länglicher Gegenstände in Körperhöhlen.

(57) Die Vorrichtung besteht aus einer rohrförmigen Hülse (1) zur Aufnahme eines festen, länglichen Gegenstandes (10) und aus einem Stössel (6) in Form eines Zylindermantels, der in Längsrichtung einen durchgehenden Schnitt (7) aufweist. Vor Gebrauch ist der Stössel (6) über die Hülse (1) geschoben, bei Gebrauch der Vorrichtung wird der Stössel (6) mit aneinanderliegenden oder sich überlappenden Rändern (7a, 7b) des Schnittes (7) in die Hülse (1) eingeschoben, um den Gegenstand (10) aus der Hülse (1) in eine Körperhöhle zu befördern.

Fig. 2

EP 0 353 185 A1

**Beschreibung**

## Vorrichtung zur Einführung fester, länglicher Gegenstände in Körperhöhlen

Die vorliegende Erfindung bezieht sich auf Vorrichtungen zur Einführung fester, länglicher, im allgemeinen zylindrischer Gegenstände, wie Tampons, Zäpfchen, Vaginalzäpfchen und dergleichen, in Körperhöhlen.

Es ist heute üblich, Monatstampons bereits in einem Applikator verpackt in den Handel zu bringen. Solche Applikatoren bestehen aus einer äusseren, rohrförmigen Hülse, in welche der Tampon eingesetzt wird, und einem ebenfalls rohrförmigen inneren Stössel, mit dessen Hilfe der Tampon durch das vordere Ende der äusseren Hülse gestossen werden kann. Solche Applikatoren sind aus Karton oder Kunststoff hergestellt und zum einmaligen Gebrauch bestimmt. Die meisten Modelle weisen den Nachteil auf, dass ihre Länge ein Mehrfaches der Länge des Tampons beträgt. Dies ist einerseits unwirtschaftlich inbezug auf Materialverbrauch, andererseits aber sind solche Applikatoren auch unpraktisch zur Unterbringung in einer Damentasche oder einer Tasche eines Kleidungsstückes.

Es wurden schon verschiedene Vorschläge gemacht, um eine Verkürzung solcher Applikatoren durch teleskopartige Anordnung des Stössels zu erzielen (zum Beispiel EP-A-223072, EP-A-252381, US-PS 4.767.773). Die Schwierigkeit, die solche Konstruktionen zu beheben versuchten, lag vor allem darin, den Tampon beim Zurückziehen des Stössels nicht ebenfalls zurück zu bewegen, was das Ausstossen des Tampons erschweren würde.

Aufgabe der vorliegenden Erfindung war die Konstruktion eines möglichst kurzen Applikators, welcher die Nachteile der bisher vorgeschlagenen Lösungen auf einfache Weise behebt.

Die Vorrichtung gemäss der vorliegenden Erfindung ist in Patentanspruch 1 definiert. Besondere Ausführungsarten sind in den abhängigen Patentansprüchen 2 bis 5 festgehalten.

Die Verwendung eines Stössels, welcher nicht aus einem geschlossenen, sondern einem in Längsrichtung offenen Mantel besteht, erlaubt es, den Stössel vor oder nach dem Einfüllen des Tampons bzw. jedes anderen festen länglichen Gegenstandes in die äussere rohrförmige Hülse, aussen über diese Hülse zu schieben. Der Stössel umgibt dann die Hülse auf einem grossen Teil ihres Umfanges mit Ausnahme des länglichen, ungedeckten Zwischenraumes, der durch die Erweiterung des Stössels entsteht. Zum Gebrauch wird der Stössel mühelos vom Aeusseren der Hülse abgezogen, leicht zusammengedrückt, um in die Oeffnung der Hülse eingeführt werden zu können, und in Form eines Rohres mit aneinanderliegenden oder überlappenden Längsrändern durch die Hülse gepresst, um den Tampon aus der Hülse zu stossen.

Die Erfindung wird hier im Detail anhand eines Ausführungsbeispiels für einen Monatstampon-Applikator beschrieben, doch ist es klar ersichtlich, dass die Vorrichtung unter Anpassung von Durchmesser und Länge ebenso zur Einführung anderer fester, länglicher Gegenstände in Körperhöhlen verwendet werden kann.

Da der vordere Teil der Hülse mit der Körperhöhle in Berührung kommt, ist es selbstverständlich, dass gewisse Bedingungen erfüllt sein müssen, wie sie von bisher bekannten Applikatoren bekannt sind. So muss das Material, aus welchem die Vorrichtung hergestellt wird, mit dem Körper und mit dem einzuführenden Gegenstand, der z.B. im Falle von Zäpfchen Medikamente enthalten kann, verträglich sein. Ferner muss er leicht zu reinigen und gegebenenfalls zu sterilisieren sein und darf keine Veränderungen beim Lagern erfahren. Die in die Körperhöhle einzuführende Spitze der Hülse wird mit Vorteil in Form von vier oder mehr blütenblätterartig geformten Lappen ausgeführt, die sich beim Durchtritt des Tampons bis auf den Durchmesser der Hülse öffnen. Eine solche Ausführungsform ist allgemein bekannt und bildet nicht Gegenstand der vorliegenden Erfindung.

Die Hülse wie auch der Stössel bestehen bevorzugt aus einem elastischen Material, wie Kunststoff, z.B. Polyäthylen oder Polypropylen, oder Papier. Die Herstellung der beiden Teile erfolgt auf üblichen Vorrichtungen und auf bekannte Weise. Ebenso kann die Hülse mit Vorteil an ihrem hinteren Ende Rippen oder andere, den Griff erleichternde Strukturierungen aufweisen. Hülse und Stössel können auf der Innenfläche mit Führungs - und Verstärkungsrippen und dergleichen versehen sein, wie dies bekannt ist. Der Stössel weist mit Vorteil mindestens dieselbe Länge wie die Hülse auf. Länge und Durchmesser der Hülse richten sich nach der Grösse des einzuführenden Gegenstandes. Für Monatstampons wird im allgemeinen eine Länge von etwa 1 cm mehr als die Tamponlänge verwendet.

Die Erfindung wird nun anhand der beiliegenden Zeichnung näher erläutert, in welcher

Fig. 1 eine leere Vorrichtung vor Gebrauch und

Fig. 2 die Vorrichtung aus Fig. 1, gefüllt mit einem einzuführenden Gegenstand und bereit zur Anwendung, zeigt.

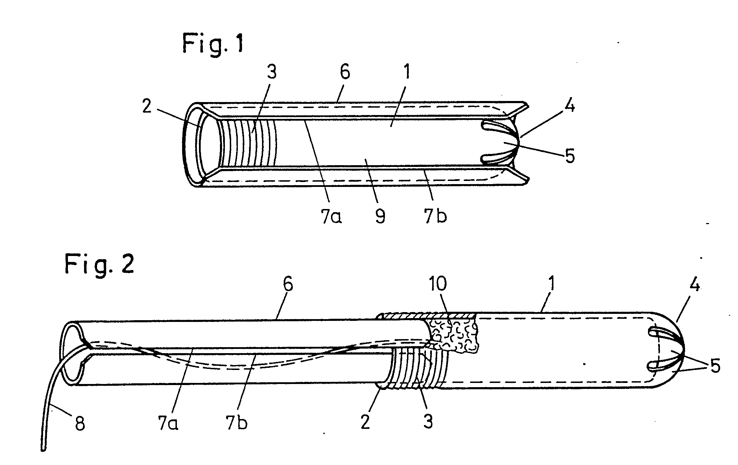

Die Hülse 1 weist im Berich des hinteren Endes 2 einige Querrillen 3 zur Erhöhung der Griffigkeit bei der Anwendung auf. Das vordere Ende 4 ist mit blütenblätterförmigen Abschnitten 5 versehen, welche dieses Ende wie eine geschlossene Blüte verschliessen. Ueber diese Hülse 1 ist ein zylindermantelförmiger Stössel 6 geschoben, der der Länge nach mit einem durchgehenden Schnitt 7 versehen ist, so dass er in Längsrichtung Ränder 7a und 7b besitzt. Wenn sich die Ränder 7a und 7b berühren, so weist der Stössel 6 einen etwas kleineren Durchmesser auf als die Hülse 1. Wie in Fig. 1 ersichtlich, liegt zwischen diesen Rändern 7a, 7b ein Zwischenraum 9, wenn der Stössel 6 über der Hülse 1 liegt.

Bei Verwendung als Applikator für Monatstampons wird ein Tampon 10 üblicherweise zuerst in die Hülse 1 eingesetzt und zwar mit dem hinteren Ende annähernd bündig zum hinteren Ende 2 der Hülse 1, bevor der Stössel 6 über die Hülse 1 geschoben wird. Die Hülse 1 weist im allgemeinen eine Länge auf, die kaum grösser als diejenige des Tampons 10 ist. die Hülse 1 muss aber lang genug sein, um teilweise in die Vagina eingeführt und leicht wieder daraus entfernt werden zu können. Der Stössel 6 kann diejenige Länge wie die Hülse aufweisen, ist aber mit Vorteil etwas länger, wie übertrieben in Fig. 1 dargestellt. Bei Verwendung der Vor richtung zur Einführung anderer länglicher Festkörper richtet sich die Länge der Hülse und Stössel nach den jeweiligen Bedürfnissen.

Die mit dem Tampon 10 gefüllte und vom Stössel 6 aussen umgebene Hülse 1, welche unter annähernd sterilen Bedingungen zusammengestellt wurde, wird zweckmässigerweise in eine eine Papierhülle oder eine geeignete luft- und feuchtigkeitsundurchlässige, gegebenenfalls durchsichtige Schutzhülle verpackt und in üblicher Anzahl zu einer Verkaufspakkung abgepackt.

Zum Gebrauch wird die von der Schutzhülle befreite Vorrichtung wie folgt betätigt : Zunächst wird ein Schnürchen 8 am hinteren Ende des Tampons 10 gelöst. Dann wird der Stössel 6 nach hinten über das hintere Hülsenende hinweg geschoben. Das Schnürchen 8 liegt nun frei im Stössel 6. Der Stössel 6, dessen Ränder 7a und 7b sich durch den Wegfall der von der etwas dickeren Hülse 1 ausgeübten Kraft wieder aneinanderliegen, wird nun von hinten in die Hülse 1 eingeführt, um den Tampon 10 durch leichte Kraft in Axialrichtung aus der Hülse 1 zu stossen, sobald die Spitze der Hülse 1 an die gewünschte Stelle der Vagina eingeführt wurde. Hülse 1 und Stössel 6 können sodann mühelos zurückgezogen werden.

In Anbetracht des günstigen Herstellungspreises der erfindungsgemässen Vorrichtung wird diese im allgemeinen als Wegwerfartikel vorgesehen. Es ist jedoch ohne weiteres möglich, beide Teile derart zu gestalten, dass sie gereinigt und wieder verwendet werden können. Dies dürfte z.B. für Suppositorien und Vaginalzäpfchen unter gewissen Umständen von Interesse sein.

**Patentansprüche**

1. Vorrichtung zur Einführung fester länglicher Gegenstände in Körperhöhlen, gekennzeichnet durch eine rohrförmige Hülse (1), deren hinteres Ende (2) offen und deren vorderes Ende (4) offen oder mittels beweglichen Abschlussteilen (5) derart teilweise geschlossen ist, dass es bei Durchtritt des Gegenstandes (10) ganz geöffnet ist, sowie durch einen Stössel (6) in Form eines Zylindermantels, der in Längsrichtung einen durchgehenden Schnitt (7) aufweist, welcher Stössel (6) vor Gebrauch der Vorrichtung über die Hülse (1) schiebbar ist unter Bildung eines Zwischenraumes (9) zwischen Rändern (7a; 7b) des Schnittes (7) und bei Gebrauch mit aneinanderliegenden oder sich überlappenden Rändern (7a; 7b) in die Hülse (1) einschiebbar ist.

2. Vorrichtung nach Patentanspruch 1, dadurch gekennzeichnet, dass das vordere Ende (5) der Hülse (1) blütenblätterförmig ausgebildet ist.

3. Vorrichtung nach Patentanspruch 1 oder 2, dadurch gekennzeichnet, dass der Stössel (6) mindestens dieselbe Länge wie die Hülse (1) aufweist.

4. Vorrichtung nach einem der Patentansprüche 1 bis 3, dadurch gekennzeichnet, dass Hülse (1) und Stössel (6) aus einem elastischen Material, insbesondere Kunststoff oder Papier, bestehen.

5. Vorrichtung nach einem der Patentansprüche 1 bis 4 zur Einführung eines Monatstampons in die Vagina, dadurch gekennzeichnet, dass ein Tampon (10) derart in der Hülse (1) angebracht ist, dass sein hinteres Ende annähernd bündig ist mit dem hinteren Ende (2) der Hülse (1).

Fig. 1

Fig. 2

<table>
<tr><td colspan="4"><img/></td></tr>
</table>

Europäisches Patentamt

EUROPÄISCHER RECHERCHENBERICHT

Nummer der Anmeldung

EP 89 81 0528

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 5) |
|---|---|---|---|
| A | US-A-4 498 899 (ETHYL MOLDED PRODUCTS CO.) * Spalte 2, Zeile 59 - Spalte 4, Zeile 5; Figuren * --- | 1-5 | A 61 F 13/32 |
| A | FR-A-2 453 637 (KAO SOAP) * Figuren 5-16; Seite 7, Zeile 20 - Seite 10, Zeile 6 * --- | 1-5 | |
| E | GB-A-2 204 495 (SMITH & NEPHEW) * Insgesamt * ----- | 1-5 | |

**RECHERCHIERTE SACHGEBIETE (Int. Cl. 5)**

A 61 F

Der vorliegende Recherchenbericht wurde für alle Patentansprüche erstellt

| Recherchenort | Abschlußdatum der Recherche | Prüfer |
|---|---|---|
| DEN HAAG | 27-10-1989 | STEENBAKKER J. |

EPO FORM 1503 03.82 (P0403)